# EUROPEAN PATENT SPECIFICATION

(11) **EP 3 024 842 B1**
(45) Date of publication and mention of the grant of the patent: **17.10.2018**
(21) Application number: 13890188.9
(22) Date of filing: 25.07.2013
(51) Int. Cl.: C07H 1/00, C07H 15/14, C07H 15/18, C07H 15/203, C07H 15/04, A61K 31/7024

(54) **PROCESS FOR THE PRODUCTION OF FONDAPARINUX SODIUM**
VERFAHREN ZUR HERSTELLUNG VON FONDAPARINUX-NATRIUM
PROCÉDÉ DE PRODUCTION DE SODIUM DE FONDAPARINUX

(43) Date of publication of application: 01.06.2016
(73) Proprietor: ScinoPharm Taiwan, Ltd., Tainan 74144 (TW)
(72) Inventor: KUO, Lung-Huang, Tainan 74144 Taiwan R.O.C. (TW); CHEN, Shang-Hong, Tainan 74144 Taiwan R.O.C. (TW); WANG, Li-Ting, Tainan 74144 Taiwan R.O.C. (TW); SHIH, Wen-Li, Tainan 74144 Taiwan R.O.C. (TW); LIAO, Yuan-Xiu, Tainan 74144 Taiwan R.O.C. (TW)
(74) Representative: Epping - Hermann - Fischer
(86) International application number: PCT/IB2013/002161
(87) International publication number: WO 2015/011517

(56) References cited:
- WO-A1-2013/115817
- US-A1- 2011 306 757
- US-A1- 2012 116 066
- US-A1- 2012 208 993
- US-A1- 2013 005 954
- US-B2- 8 288 515
- LIN FENG ET AL: "Synthesis of Fondaparinux: modular synthesis investigation for heparin synthesis", CARBOHYDRATE RESEARCH, PERGAMON, GB, vol. 371, 16 January 2013 (2013-01-16), pages 32-39, XP028527227, ISSN: 0008-6215, DOI: 10.1016/J.CARRES.2013.01.003
- ANNA KOZIOL ET AL: "A Fast and Effective Hydrogenation Process of Protected Pentasaccharide: A Key Step in the Synthesis of Fondaparinux Sodium", ORGANIC PROCESS RESEARCH AND DEVELOPMENT, vol. 17, no. 5, 17 May 2013 (2013-05-17), pages 869-875, XP055314997, US ISSN: 1083-6160, DOI: 10.1021/op300367c
- ICHIKAWA YOSHITAKA ET AL: "Synthetic studies on mucopolysaccharides. Part III. Synthesis, from cellobiose of a trisaccharide closely related to the GlcNAc .fwdarw. GlcA .fwdarw. GlcN segment of the antithrombin-binding sequence of heparin", CARBOHYDRATE RESEARCH, PERGAMON, GB, vol. 141, no. 2, 1 January 1985 (1985-01-01), pages 273-282, XP002539721, ISSN: 0008-6215, DOI: 10.1016/S0008-6215(00)90458-0

## Description

### CROSS-REFERENCES TO RELATED APPLICATIONS

Not applicable

### BACKGROUND OF THE INVENTION

Fondaparinux sodium (CAS 114870-03-0) is a member of oligosaccharides / heparins with a chemical name of O-[2-Deoxy-6-O-sulfo-2-(sulfoamino)-alpha-D-glucopyranosyl]-(1--4)-O-(beta-D-glucopyranurosonyl)-(1--4)-O-[2-deoxy-3,6-di-O-sulfo-2-(sulfoamino)-alpha-D-glucopyranosyl]-(1--4)-O-(2-O-sulfo-alpha-L-idopyranurosonyl)-(1--4)-O-[2-deoxy-1-O-methyl-6-0-sulfo-2-(sulfoamino)-alpha-D-glucopyranoside] decasodium salt, which developed by Choay, S.A. (see US 4,818,816). The compound is a synthetic pentasaccharide Factor Xa inhibitor which is indicated as an anticoagulant drug used for the prevention of deep vein thrombosis in patients who have had orthopedic surgery as well as for the treatment of deep vein thrombosis and pulmonary embolism. It was approved by the United States Food and Drug Administration in 2001, marketed under the trade name Arixtra™ which is administrated subcutaneously.

The preparation process of Fondaparinux sodium disclosed in U.S. Patent No. 4,818,816 is unsuitable for a large scale production since this process takes over 60 steps to afford a final product with low yield.

U.S. Patent No. 8,288,515 applies protection and de-protection steps to prepare Fondaparinux sodium. However, the de-protection step results in low yields and consumes additional reaction time.

Another process is disclosed in U.S. 2011/0306757, but the additional reduction step of an azide needs further purification and the final N-sulfonation step remains in low yield (68%).

US 2012/0116066 describes the preparation of Fondaparinux sodium and its intermediates. However, the preparation of some intermediates such as EMod3 needs column purification. Moreover, the low α/β ratios in the coupling between C monomer and D monomer as well as numerous time-consuming procedures are not optimal.

In view of the above, there is still a need for a simple process with higher yield/purity for industrial preparation of Fondaparinux sodium.
US 2012/0208993 A1 relates to intermediates, and processes for the chemical synthesis of AT-III binding heparin or heparinoid, pentasaccharides.
Lin Feng et al.: "Synthesis of Fondaparinux: modular synthesis investigation for heparin synthesis", Carbohydrate Research, Pergamon, GB, vo. 371, 16 January 2013, pages 32-39 discloses a method of glycosylation to yield Compound 27 (i.e., the trisaccharide ABC1) in toluene without indicating the α/β ratio of the product.
Anna Koziol et al.: "A Fast and Effective Hydrogenation Process of Protected Pentasaccharide: A Key Step in the Synthesis of Fondaparinux Sodium", Organic Process Research and Development, vol. 17, no. 5, 17 May 2013, pages 869-875 discloses the conversion of ABCDE1 into ABCDE5 making use of 4-methoxybenzyl (Mpm) to protect the primary hydroxyl groups.

### BRIEF SUMMARY OF THE INVENTION

The present invention provides an economic process to prepare Fondaparinux sodium. This object is achieved by providing a process for preparing Fondaparinux sodium according to claim 1. Preferred embodiments are set forth in the dependent claims.

### BRIEF DESCRIPTION OF THE DRAWINGS

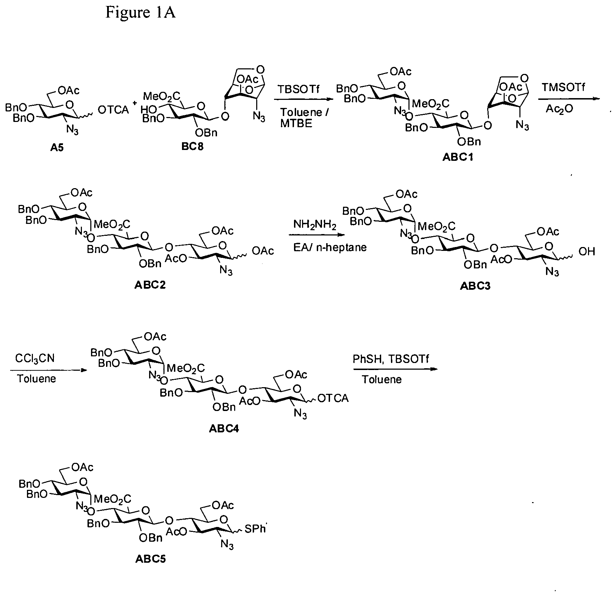

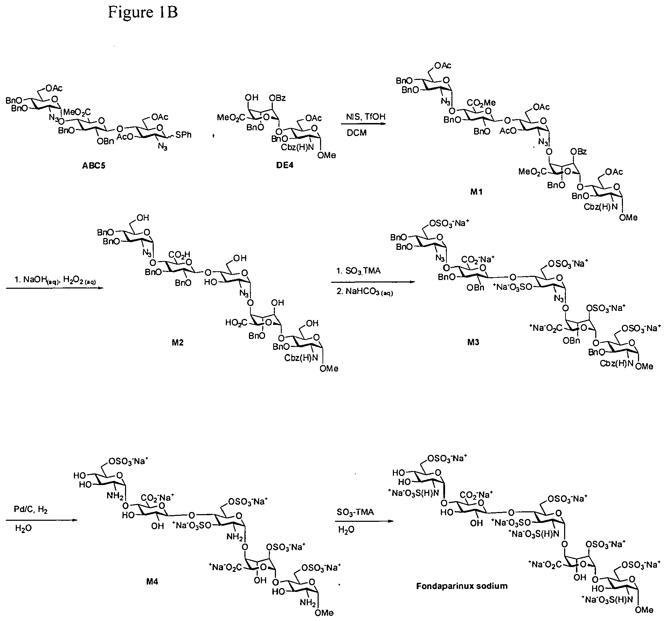

**Figures 1A** **and** **1B** show an improved synthetic route for Fondaparinux sodium according to the present invention employing methods provided herein.

### DETAILED DESCRIPTION OF THE INVENTION

### I. General

The present invention provides a process for preparation of Fondaparinux sodium. The novel processes have been discovered to be of higher yield and with reduced impurity. The process provided herein also reduces the time required to complete numerous transformations (synthetic steps).

### II. Definitions

As used herein, the term "contacting" refers to the process of bringing into contact at least two distinct species such that they can react. It should be appreciated, however, that the resulting reaction product can be produced directly from a reaction between the added reagents or from an intermediate from one or more of the added reagents which can be produced in the reaction mixture.

As used herein, the term "alkyl" by itself or as part of another substituent, means, unless otherwise stated, a straight or branched chain hydrocarbon radical. Alkyl substituents, as well as other hydrocarbon substituents, may contain number designators indicating the number of carbon atoms in the substituent (i.e., C₁-C₈ means one to eight carbons), although such designators may be omitted. Unless otherwise specified, the alkyl groups of the present invention contain 1 to 12 carbon atoms. For example, an alkyl group can contain 1-2, 1-3, 1-4, 1-5, 1-6, 1-7, 1-8, 1-9, 1-10, 1-11, 1-12, 2-3, 2-4, 2-5, 2-6, 3-4, 3-5, 3-6, 4-5, 4-6 or 5-6 carbon atoms. Examples of alkyl groups include methyl, ethyl, n-propyl, isopropyl, n-butyl, t-butyl, isobutyl, sec-butyl, n-pentyl, n-hexyl, n-heptyl, n-octyl, and the like.

As used herein, the term 'substituted' when refering to alkyl, phenyl and benzyl, refers to one or more substituents, typically one to three substituents that are selected to be non-interfering substituents such as halogen, amino, hydroxy, nitro, cyano, lower alkyl (e.g., C₁₋₄ alkyl), lower alkoxy (e.g., C₁₋₄ alkyl-O-), lower alkylamino (e.g., C₁₋₄ alkyl-NH-), di-lower alkylamino (e.g., di-C₁₋₄ alkylamino), and haloalkyl. One of skill in the art will appreciate that additional substituted alkyl, phenyl and benzyl are known and useful in the context of the invention.

As used herein, a solvent mixture may comprise a percentage of a first solvent in a second solvent. Unless otherwise stated, the percentage is by volume.

Various protecting groups and protecting reagents, including hydroxyl protecting reagents, are well known to one of ordinary skill in the art and include compounds that are disclosed in Protective Groups in Organic Synthesis, 4th edition, T. W. Greene and P. G. M. Wuts, John Wiley & Sons, New York, 2006, which is incorporated herein by reference in its entirety.

### III. Embodiments of the Invention

In one aspect, the provided herein is a process for the preparation of Fondaparinux sodium of formula ABCDE5 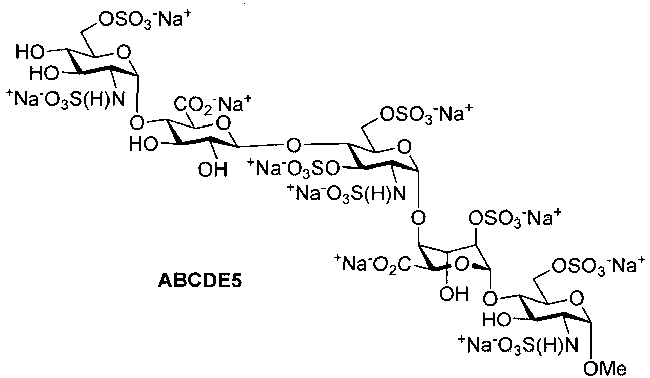 comprising contacting a compound of formula **ABC5** 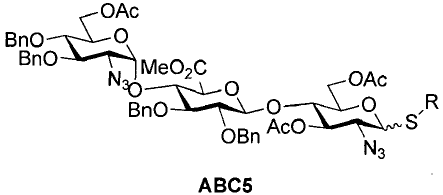 with a compound of formula **DE4** 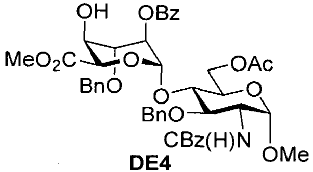 to obtain a compound of formula **ABCDE1** 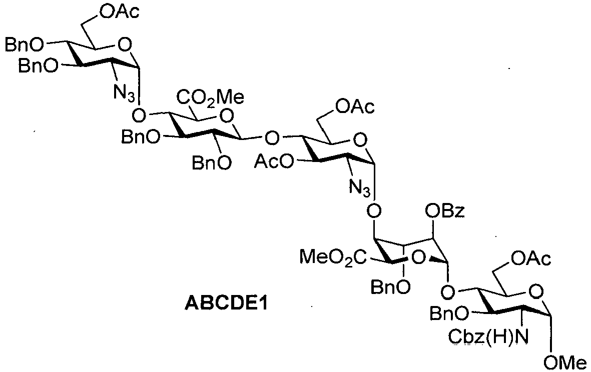 and then converting the compound of formula **ABCDE1** to Fondaparinux sodium;
wherein R is selected from the group consisting of alkyl, phenyl, benzyl, substituted alkyl, substituted phenyl, substituted benzyl.

The conversion of **ABCDE1** to Fondaparinux sodium is described in more detail below.

In one group of embodiments, **ABC5** is obtained using a process comprising converting a compound of formula **ABC4** 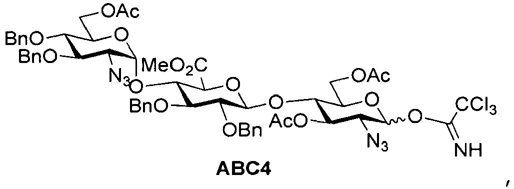 in the presence of a promoter and a thiol to provide the compound of formula **ABC5.** The promoters used in this group of embodiments are selected from the group consisting of TESOTf, TMSOTf, TfOH, TBSOTf and mixtures thereof.

In another group of embodiments, **ABC4** is prepared from a process comprising:
a) converting a compound of formula **A4** 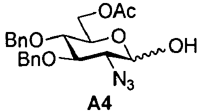 to provide a compound of formula **A5** 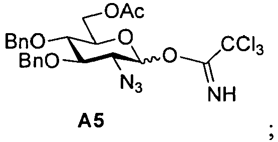
b) contacting the compound of formula **A5** with a compound of formula **BC8** 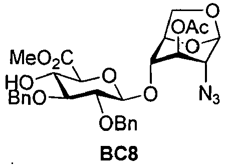 under conditions sufficient to provide a compound of formula **ABC1** 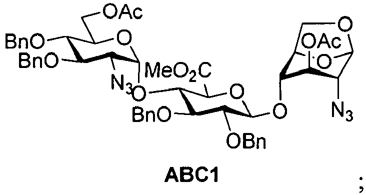 and
c) converting the compound of formula **ABC1** to provide the compound of formula **ABC4**

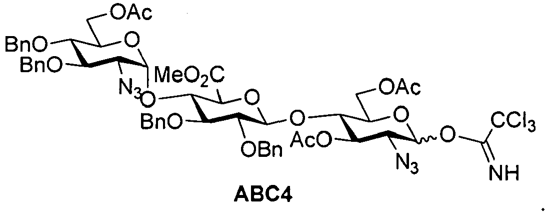

In step (a) above, the conversion of **A4** to **A5** is conducted in the presence of a base and trichloroacetonitrile. In one group of embodiments the base is an organic amine (e.g., DBU, pyridine, triethylamine, diisopropylethyl amine, pyrrolidine; or any other such organic base). In another group of embodiments, the base is an inorganic base (e.g., potassium carbonate, sodium carbonate, potassium bicarbonate, sodium bicarbonate, cesium carbonate, potassium phosphate, or any other such inorganic base). A number of bases are useful in this conversion, particularly DBU, potassium carbonate and mixtures thereof. Preferably the base used is an alkali base.

In step (b) above, the contacting of **A5** with a compound of formula **BC8** to provide a compound of formula **ABC1** will generally take place in an organic solvent in the presence of a promoter. A variety of solvents are useful such as ether (e.g., diethyl ether, tetrahydrofuran), MTBE, IPE, diglyme, toluene, DCM, DCE and mixtures thereof. In one group of embodiments, the solvent is selected from diethyl ether, MTBE, IPE, diglyme, toluene, DCM and mixtures thereof. In one group of embodiments, the solvent is a mixture of 0-20% toluene or DCM in MTBE. In other embodiments, the solvent is a mixture of about 15-25% toluene in MTBE, more preferably about 20% toluene in MTBE. As with the above conversion of **ABC4** to **ABC5,** the promoters used in this group of embodiments are selected from the group consisting of TESOTf, TMSOTf, TfOH, TBSOTf and mixtures thereof.

In step (c) above, the conversion of **ABC1** to a compound of formula **ABC4** will generally take place via a sequence of steps as follows. (c-1) Initially **ABC1** is converted to a ketal-hydrolysed product **ABC2** in the presence of a promoter, an organic solvent, a base and an acylating agent. Generally the reactions are carried out at about ambient temperature (e.g., from 20 °C to 30 °C), optionally at elevated temperatures. Suitable promoters include trialkylsilyls, trifluoromethanesulfonates, and mixtures of trialkylsilyls and trifluoromethanesulfonates. An exemplary ketal hydrolysis and anomeric acylation is provided in Example 2. (c-2) The acetyl group at the anomeric position in **ABC2** is cleaved in the presence of a base and an aprotic solvent to provide compound **ABC3.** Examples of aprotic solvents include toluene, xylenes, THF, EA, DCM, DCE and the like. An exemplary acetyl group cleavage is described in Example 3. (c-3) A leaving group is introduced at the anomeric position of **ABC3** to provide compound **ABC4.** Examples of suitable leaving groups include halogens, activated esters, acetimidates or the like. Generally the reaction is carried out in an aprotic solvent. Examples of aprotic solvents include toluene, xylenes, THF, EA, DCM, DCE and the like. An exemplary introduction of a trichloroacetimidate group (TCA) leaving group is provided in Example 4.

In another aspect, provided herein are novel intermediates having the formula **ABC5:** 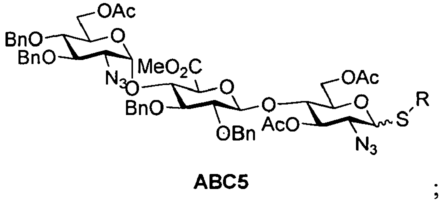 wherein R is selected from the group consisting of alkyl, phenyl, benzyl, substituted alkyl, substituted phenyl and substituted benzyl. In one group of embodiments, a compound of formula **ABC5** described herein has the formula **ABC5a:**

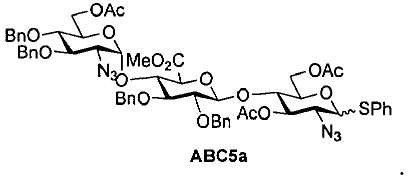

In another group of embodiments, R in **ABC5** is substituted phenyl. In yet another group of embodiments, R in **ABC5** is benzyl or substituted benzyl. In further embodiments, R in **ABC5** is alkyl or substituted alkyl.

Also provided herein is a process for preparing Fondaparinux sodium comprising:
i) contacting the compound of formula **A5** 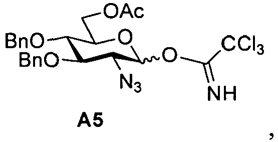 with the compound of formula **BC8** 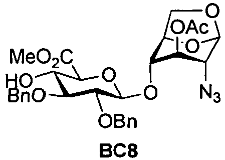 to provide the compound of formula **ABC1** 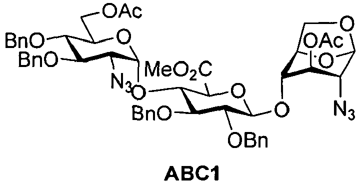 in a mixture of toluene/MTBE; and
ii) converting the compound of formula **ABC1** to provide Fondaparinux of formula **ABCDE5:**

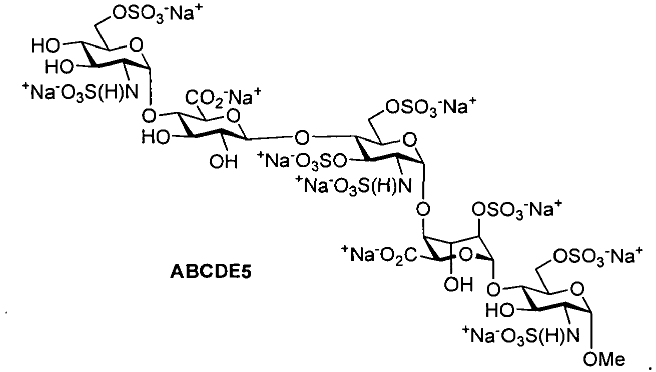

In one group of embodiments, step (i) above is conducted in toluene/MTBE in the presence of a base. In certain embodiments, the base is an organic amine (e.g., DBU, pyridine, triethylamine, diisopropylethyl amine, pyrrolidine, or any other such organic base). In another group of embodiments, the base is an inorganic base (e.g., potassium carbonate, sodium carbonate, potassium bicarbonate, sodium bicarbonate, cesium carbonate, potassium phosphate, or any other such inorganic base).

In step (ii) above, the conversion of **ABC1** to **ABCDE5** is achieved via a series of reactions as follows. (ii-1) Initially **ABC1** is converted to a ketal-hydrolysed product **ABC2** in the presence of a promoter, an organic solvent, a base and an acylating agent. Generally the reactions are carried out at about ambient temperature (e.g., from 20 °C to 30 °C), optionally at elevated temperatures. Suitable promoters include trialkylsilyls, trifluoromethanesulfonates, and mixtures of trialkylsilyls and trifluoromethanesulfonates. An exemplary ketal hydrolysis and anomeric acylation is provided in Example 2. (ii-2) The acetyl group at the anomeric position in **ABC2** is cleaved in the presence of a base and an aprotic solvent to provide compound **ABC3.** Examples of aprotic solvents include toluene, xylenes, THF, EA, DCM, DCE and the like. An exemplary acetyl group cleavage is described in Example 3. (ii-3) A leaving group is introduced at the anomeric position of **ABC3** to provide compound **ABC4.** Examples of suitable leaving groups include halogens, activated esters, acetimidates or the like. Generally the reaction is carried out in an aprotic solvent. Examples of aprotic solvents include toluene, xylenes, THF, EA, DCM, DCE and the like. An exemplary introduction of a trichloroacetimidate group (TCA) leaving group is provided in Example 4. (ii-4) A thio-donor compound **ABC5** is generated from **ABC4** by reaction of **ABC4** with a thiol in the presence of a promoter in an organic solvent. Generally the reaction is carried out in an aprotic solvent. Examples of aprotic solvents include toluene, xylenes, THF, EA, DCM, DCE and the like. Suitable promoters include trialkylsilyls, trifluoromethanesulfonates, and mixtures of trialkylsilyls and trifluoromethanesulfonates. An exemplary introduction of a thiophenyl group is described in Example 4. Generally the reaction mixture includes a base. Examples of bases include organic bases such as triethylamine, diisopropylamine, diisopropylethylamine and the like, or inorganic bases such as potassium carbonate, sodium carbonate, cesium carbonate and the like. One of skill in the art will understand that the introduction of a thio-donor moiety is possible under various conditions and depends on the leaving group present in the compound. (ii-5) The thio donor compound **ABC5** is reacted with an acceptor compound such as **DE4** to obtain an oligosaccharide **ABCDE1.** The reaction is carried out in the presence of a radical initiator and/or a promoter in an organic solvent. Generally the reaction is carried out in an aprotic solvent. Examples of aprotic solvents include toluene, xylenes, THF, EA, DCM, DCE and the like. The reaction is generally carried out at a temperatures ranging from about -30 °C to about 40 °C. Suitable promoters include trialkylsilyls, trifluoromethanesulfonates, and mixtures of trialkylsilyls and trifluoromethanesulfonates. Non-limiting examples of radical initiators include N-iodosuccinimide, N-bromosuccinimide and the like. An exemplary reaction between a donor and an acceptor compound is shown in Example 5. One of skill in the art will understand that the donor-acceptor reaction is possible under various conditions and depends on the thio-donor moiety and the acceptor moiety present in the compounds.

The conversion of **ABCDE1** to **ABCDE5** is achieved as follows. (ii-6) The ester group in **ABCDE1** is cleaved in the presence of a peroxide and a base in an aprotic solvent to provide **ABCDE2.** Examples of aprotic solvents include toluene, xylenes, THF, EA, DCM, DCE and the like. The reaction is generally carried out initially at temperatures below 10 °C, then warmed to ambient temperature (e.g., 20 °C to 30 °C). Example 6 provides an exemplary procedure for ester cleavage in an oligosaccharide. (ii-7) **ABCDE2** is then O-sulfated in the presence of a base to provide **ABCDE3.** The reaction is generally carried out in an aprotic solvent by introduction of sulfate groups using a sulfating reagent, followed by addition of a base to introduce counterions for the sulfate groups. Example 7 provides an exemplary procedure for introduction of sodium sulfate groups. (ii-8) The Cbz protecting group in **ABCDE3** is removed under suitable conditions to provide **ABCDE4.** In some cases, hydrogenation is used which also reduces the azido groups to amine groups. The hydrogenation is typically carried out at ambient temperatures (e.g., 20 °C to 30 °C) for a period of 1-5 days, preferably 1-3 days. Example 8 provides an exemplary procedure for conversion of **ABCDE3** to **ABCDE4.** (ii-9) **ABCDE4** is converted to Fondaparinux via an N-sulfation step, using a sulfating reagent, followed by addition of a base to introduce counterions for the sulfate groups. The compound is then desalted. Example 9 provides an exemplary procedure for introduction of sodium sulfate groups. The sodium salt is desalted in the final step to obtain Fondaparinux.

In a select group of embodiments, a solvent mixture for the reaction of **A5** with **BC8** in step (i) above is a toluene/MTBE mixture having a ratio of toluene/MTBE from 10% to 30%, or from 15% to 25%, preferably 20%.

### EXAMPLES

The following examples are presented to describe the invention in further detail. However, the present invention is by no means restricted to the specific embodiments described herein. The following abbreviations are used in the specification, and examples: DCM is dichloromethane; EA is ethyl acetate; THF is tetrahydrofuran; MTBE is methyl *tert*-butyl ether; DMAc is dimethylacetamide; OTCA is a trichloroacetimidate group; DCE is dichloroethane; IPE is isopropyl ether; CBz is carboxybenzyl, a carbamate protecting group. _Compound **BC8** can be prepared according to U.S. application publication no. 20120083594. Compound **A4** can be prepared according to procedures in J. Am Chem Soc., 2005, 127, 3767-3773; or Tetrahedron: Asymmetry, 2005, 16(2), 411 - 424.

### Example 1

### Preparation of ABC1

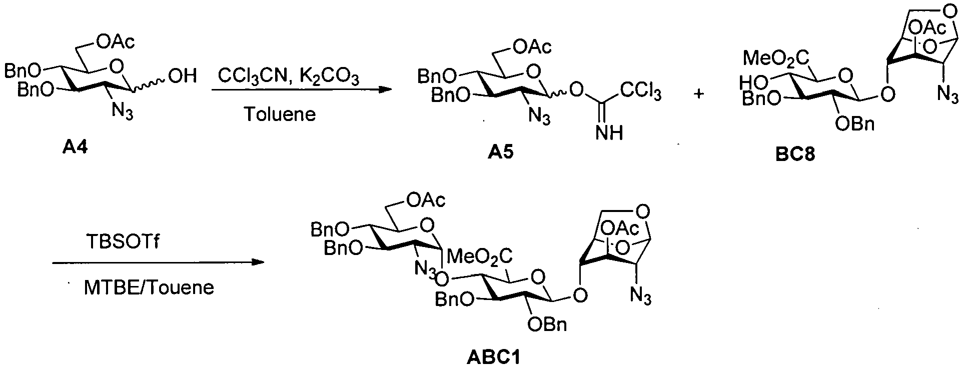

### A4 to A5

A four-necked round bottom flask was equipped with a mechanical stirrer and a thermometer. To the flask was added **A4** (32 g, 75 mmol, 1.4 equiv), toluene (64 mL), K₂CO₃ (52 g, 374 mmol, 7.0 equiv), and CCl₃CN (37 mL, 374 mmol, 7.0 equiv) at 20-30°C under nitrogen. The mixture was stirred at 20-30°C for 4 hr. The mixture was filtered and the filtered cake was washed with toluene (64 mL). The filtrate and washing were combined to afford **A5** in toluene solution. After being cooled to no more than -10°C, the **A5**/toluene solution was ready to be used.

### BC8 to ABC1

A four-necked round bottom flask was equipped with a mechanical stirrer and a thermometer. To this flask was added **BC8** (32 g, 53 mmol, 1 equiv) and MTBE (576 mL) at 20-30°C under nitrogen. The mixture was heated to no more than 45°C for dissolution. After being cooled to 20-30°C, 3Å molecular sieves (15 g) were added to the mixture and the resulting mixture was stirred at this temperature for 2 hr. The mixture was then cooled to -35 to -25°C. TBSOTf (5 mL, 21 mmol, 0.4 equiv) was added at -35 to -25°C, and the mixture was stirred at this temperature for about 15 min. The resulting mixture containing **BC8** and 3Å molecular sieves in MTBE was ready to be used.

To the flask containing **A5**/toluene solution was added into the mixture containing **BC8** and 3Å molecular sieves in MTBE over 30 min while maintaining temperature at -35 to -25°C. The mixture was stirred at -35 to -25°C for 1 hr. Triethylamine (23 mL, 160 mmol, 3 equiv) and Ac₂O (5 mL, 53 mmol, 1 equiv) were successively added at -35 to -25°C. The mixture was heated to about 50°C and stirred for 6 hr. The mixture was filtered and the filtered cake was washed with MTBE (64 mL). The filtrate and washing were combined and concentrated to afford crude **ABC1** solution. Crude **ABC1** solution was purified using column chromatography (silica gel; eluting solvent: EtOAc/ n-heptane(first eluting solvent is 1:4 and then 2:3)) and then concentrated to afford **ABC1** solution (50 g, 88%) in EtOAc /n-heptane (1/1(v/v)).

### Example 2

### Preparation of ABC2

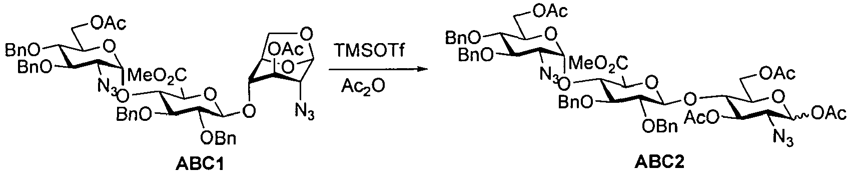

A three-necked round bottom flask was equipped with a mechanical stirrer and a thermometer. To the flask was added the previously reserved **ABC1** in EtOAc / n-heptane solution (162mL, 1/1(v/v)) at 20-30°C under nitrogen. After the mixture was cooled to 0-10°C, Ac₂O (16.3 g, 0.16 mol, 3.0 equiv) and TMSOTf (3.6 g, 0.02 mol, 0.3 equiv) were successively added at this temperature. The mixture was stirred at 0-10°C for not less than 10 hr. Triethylamine (45 mL, 0.27 mol, 6.0 equiv) was slowly added at 0-10°C. The mixture was stirred at 0-10°C for 1 hr. 20% NaCl_{(aq)} (64 mL, 2 vol) was slowly added at 0-10°C. The mixture was stirred for 2 hr. The separated aqueous portion was discarded. The separated organic portion containing **ABC2** in EtOAc / n-heptane (1/1 (v/v)) solution was ready to be used in the next step.

### Example 3

### Preparation of ABC3

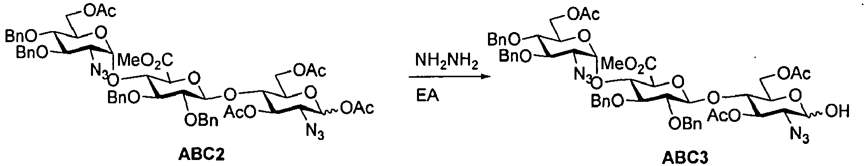

A three-necked round bottom flask was equipped with a mechanical stirrer and a thermometer. To the flask was added the previously reserved **ABC2** in EtOAc / n-heptane (1/1 (v/v)) solution at 20-30°C under nitrogen. H₂NNH₂-H₂O (3.8 g, 80 mmol, 1.4 equiv) was added at 20-30°C, and the mixture was stirred at this temperature for 3 hr. A 5% solution of NaCl_{(aq)} (160 mL) was added at 20-30°C, and the mixture was stirred at this temperature for 1 hr. The stirring was stopped for phase separation. The separated aqueous phase was discarded. The organic and emulsion portions were combined and concentrated to afford crude **ABC3** in EtOAc / n-heptane solution. Crude **ABC3** solution was purified with column chromatography (silica gel; eluting solvent: acetone/ toluene (containing 0.05%(v/v) of Et₃N, 5/95(v/v))) and then concentrated to afford **ABC3** in toulene solution (44 g, 94%).

### Example 4

### Preparation of ABC5

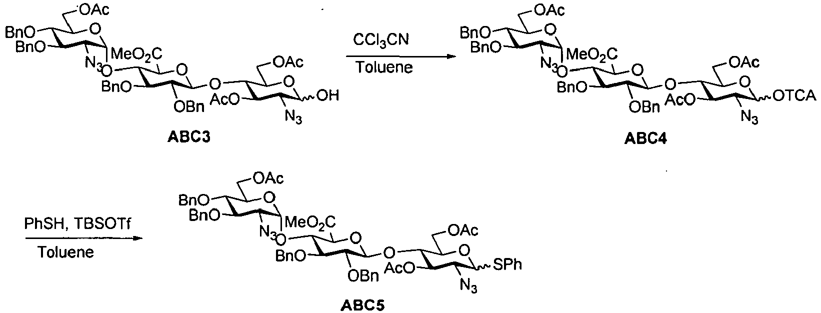

### ABC3 to ABC4

A four-necked round bottom flask was equipped with a mechanical stirrer and a thermometer. To the flask was added **ABC3**/toluene solution (about 96 mL, 3 vol) at 20-30°C under nitrogen. K₂CO₃ (74 g, 0.53 mol, 10 equiv) and CCl₃CN (77 g, 0.53 mol, 10 equiv) were successively added at 20-30°C. The mixture was stirred at 20-30°C for not less than 4 hr. The mixture was filtered and the filtered cake was washed with toluene (64 mL, 2 vol). The filtrate and washing were combined to afford **ABC4** in toluene solution. After being cooled to no more than -5°C, the **ABC4**/toluene solution (about 160 mL, 5 vol) was ready to be used.

### ABC4 to ABC5

A four-necked round bottom flask was equipped with a mechanical stirrer and a thermometer. To the flask was added thiophenol (24 g, 0.2 mmol, 4 equiv) and toluene (260 mL) at 20-30°C under nitrogen. The mixture was cooled to -20 to -10°C. TBSOTf (21 g, 0.08 mol, 1.5 equiv) was added at -20 to -10°C. The resulting mixture containing thiophenol and TBSOTf in toluene was ready to be used.

To the flask containing **ABC4** solution was added the mixture containing thiophenol and TBSOTf in toluene over 30 min while maintaining temperature at -20 to -10°C. The mixture was stirred at -20 to -10°C for 2 hr. Et₃N/toluene (15 mL/65 mL) was slowly added over about 30 min while maintaining temperature no more than -5°C. The mixture was stirred at no more than -5°C for 30 min. The mixture was concentrated to afford crude **ABC5** solution in toluene. **ABC5** solution was purified with column (silica gel; eluting solvent: EtOAc/ toulene (containing 0.05% (v/v) of Et₃N, 2/98, (v/v))) to afford **ABC5** in toulene solution (42 g, 88%).

### Example 5

### Preparation of ABCDE1

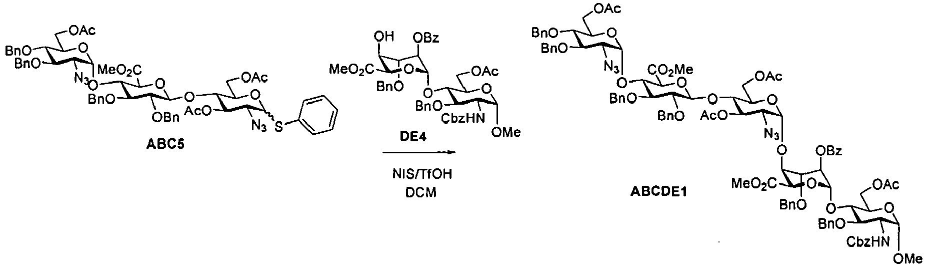

**ABC5** (35 g, 0.03 mol, 1.0 equiv), **DE4** (28 g, 0.033 mol, 1.1 equiv), and DCM (700 g,) were added into a four-necked round bottom flask equipped with a mechanical stirrer and a thermometer at 20-40°C under nitrogen. The mixture was stirred at 20-40°C for 30 min to obtain a homogeneous solution. 3Å molecular sieves (35 g) was added at 20-40°C, and the mixture was stirred at this temperature for 1 hr.

After the mixture was cooled to -30 to -20°C, N-iodo-succinimide (NIS) (10.2 g, 1.5 equiv, 0.045 mol) was added at this temperature and stirred for 15 min. TfOH (1.8 g, 0.012 mol, 0.4 equiv) in DCM (10 mL) was slowly added at -30 to -20°C, and the mixture was stirred at this temperature for 2 hr. Et₃N (6.1 g, 0.06 mol, 2 equiv) was added at -30 to -20°C, and the mixture was stirred at this temperature for 30 min. The mixture was filtered through a celite pad, and the filtered cake was washed with DCM (140 mL). The combined filtrate and washing was added 30% Na₂S₂O₃•5H₂O_{(aq)} (105 mL, 3 vol) at 20-40°C. After the mixture was stirred at 20-40°C for 1 hr, the stirring was stopped for about 5 min to effect phase separation. The separated aqueous portion was discarded. The separated organic portion was concentrated to afford crude **ABCDE1** solution in DCM. Crude **ABCDE1** solution was purified with column chromatography (silica gel; eluting solvent: EtOAc/ toulene (containing Et₃N (0.1% (v/v)) 1/9(v/v)) to provide a solution of **ABCDE1** in toulene solution.

**ABCDE1** in toulene solution (about 105 mL) was added into a four-necked round bottom flask equipped with a mechanical stirrer and a thermometer under nitrogen. After the mixture was heated to 35-45°C, IPA (105 mL) and *n*-heptane (105 mL) were sequentially added at this temperature. **ABCDE1** seed (0.035 g) was added at 35-45°C, and the mixture was stirred at this temperature for 1 hr. After *n*-heptane (175 mL) was added at 35-45°C, the mixture was cooled to 15-25°C and stirred for 1hr. The mixture was filtered and the filtered cake was washed with *n*-heptane (70 mL). The wet cake was dried at no more than 60°C to afford **ABCDE1** (39 g, 65%) as a white solid.

### Example 6

### Preparation of ABCDE2

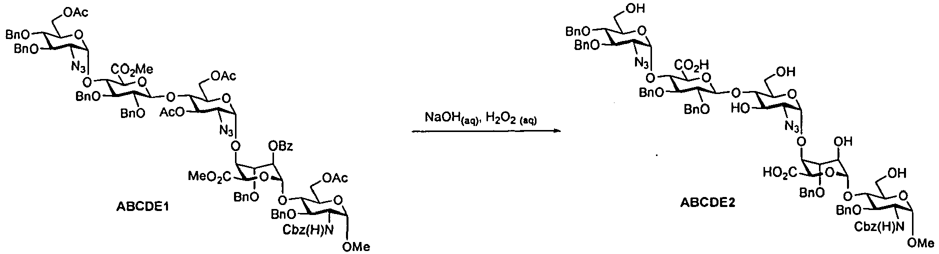

THF (250 mL) and **ABCDE1** (50 g, 26.4 mmol, 1.0 equiv) were charged into a four-necked round bottom flask at 20-40°C under nitrogen. The mixture was cooled to 10°C, and 35% H₂O_{2(aq)} (102.5 mL, 1161 mmol, 44 equiv) was added at this temperature. 2N NaOH_{(aq)} (356 mL, 712.4 mmol, 27 equiv) was added at 10°C. The mixture was heated to 20-30°C and stirred for 48 hr. The stirring was stopped for about 5 min to affect phase separation. The separated organic portion was saved, and the separated aqueous portion was discarded. The reserved organic portion was added 30% Na₂S₂O₃•5H₂O_{(aq)} (250 mL, 5 vol), and the mixture was stirred for about 5 min. The stirring was stopped for about 5 min to affect phase separation. The separated organic portion was saved, and the separated aqueous portion was discarded. The reserved organic portion was added 30% Na₂S₂O₃•5H₂O_{(aq)} (250 mL, 5 vol), and the mixture was stirred for about 5 min. The stirring was stopped for about 5 min to affect phase separation. The separated organic portion was saved, and the separated aqueous portion was discarded. The reserved organic portion was added H₂O (500 mL, 10 vol), and IN HCl_{(aq)} (45 mL, 0.9 vol) was added till pH of the mixture reached 4-5. Acetone (250 mL, 5 vol) was added and the mixture was concentrated at 35-60°C to a volume of about 700 mL. IN HCl_{(aq)} (5 mL) was added to reach a pH of the mixture of about 2.5-3.5. After being stirred at 20-30°C for 30 min, the mixture was filtered and the filtered cake was washed with H₂O (250 mL). The wet cake was dried at no more than 60°C to afford **ABCDE2** as white solid (38.4 g, 82% yield).

### Example 7

### Preparation of ABCDE3

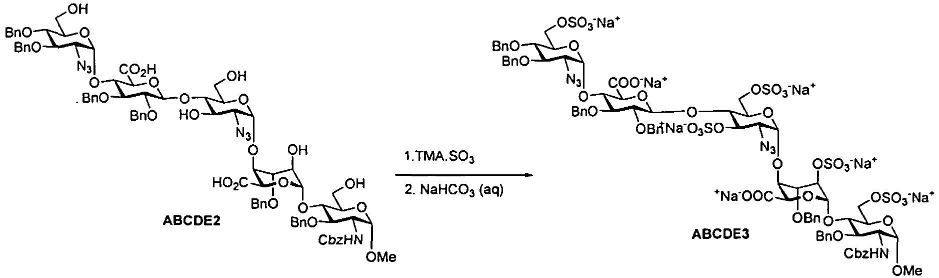

**ABCDE2** (8 g, 1.0 equiv, 5.02 mmol), SO₃-TMA complex (38.4 g, 55 equiv, 275.92 mmol), and DMAc (88 mL) were added into a round bottom flask equipped with a mechanical stirrer and a thermometer under nitrogen at 20-40°C. The slurry mixture was heated to 55-65°C and stirred for 6 hr. After being cooled to no more than 10°C, to the mixture was added 8% NaHCO_{3(aq)} (40 mL) at no more than 30°C_{.} The mixture was filtered and the filtered cake was washed with DMAc (96 mL). After the combined filtrate and washing was cooled to no more than 10°C, water(88 mL) was slowly added while maintaining temperature at 30°C. A mixture containing crude **ABCDE3** solution DMAc/ water was thus obtained. **ABCDE3** was purified with HP20SS resin by eluting solvent via NaCl_{(aq)} (10%) and then MeOH and then solvent exchanged by water to afford **ABCDE3** aqueous solution.

### Example 8

### Preparation of ABCDE4

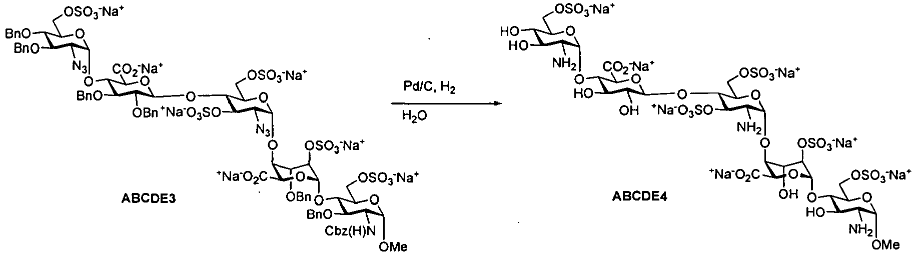

**ABCDE3** aqueous solution (based on 8 g of **ABCDE2),** and 10% Pd/C (3.2 g, 40% wt) were added into an autoclave at 20-30°C. The mixture was exposed to hydrogen (0-0.5 kg, gauge pressure) at 20-30°C for 48 hr. The mixture was filtered through a celite pad, and the filtered cake was washed with water (32 mL). After the combined filtrate and washing was added activated charcoal (1.6 g,) at 20-30°C, the mixture was stirred at this temperature for 3 hr. The mixture was filtered through a celite pad, and the filtrate was saved. The reactor was rinsed with PPW (32 mL), and the solution was filtered through a 0.2 micrometer filter. The two filtrates were combined to afford a **ABCDE4** aqueous solution.

### Example 9

### Preparation of Fondaparinux

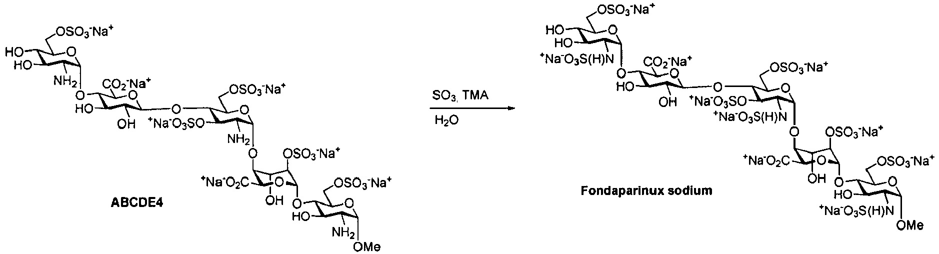

**ABCDE4** aqueous solution (based on 8 g of **ABCDE2**) was added into a round bottom flask equipped with a mechanical stirrer and thermometer at 20-40°C. The mixture was added IN HCl_{(aq)} till pH reached 8-9. After SO₃•TMA (23.04 g, 33 equiv, 165.5 mmol) was added at 20-40°C, the mixture was heated to 40-50°C and stirred for 10 hr. The mixture was cooled to no more than 10°C. The mixture was filtered and the filtered cake was washed with water (32 mL). The filtrate was added IN NaOH_{(aq)} till pH reached 9-10. The mixture was heated to 45-55°C and stirred for 20 hr. The mixture was cooled to no more than 30°C. A mixture containing crude Fondaparinux sodium aqueous solution was thus obtained.

Crude Fondaparinux sodium aqueous solution (2.4 g) was purified with Q Sepharose Fast Flow resin (QSFF) (190 mL) using the eluting solvent via 0.4M NaCl_{(aq)}, 0.8M NaCl_{(aq)} and 2M NaCl_{(aq)} to afford Fondaparinux sodium solution. Fondaparinux sodium was desalted by 0.1 m² of 1 kDa regenerous cellulose (RC) membrane using Tangential Flow Filtration (TFF) and then lyophilized to afford Fondaparinux (2.2 g, 80%).

## Claims

1. A process for preparing Fondaparinux sodium comprising:
i) contacting the compound of formula **A5** 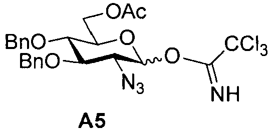 with the compound of formula **BC8** 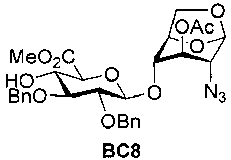 to provide the compound of formula **ABC1** 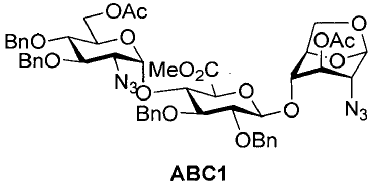 in a mixture of toluene/MTBE; and
ii) converting the compound of formula **ABC1** to Fondaparinux of formula **ABCDE5:**

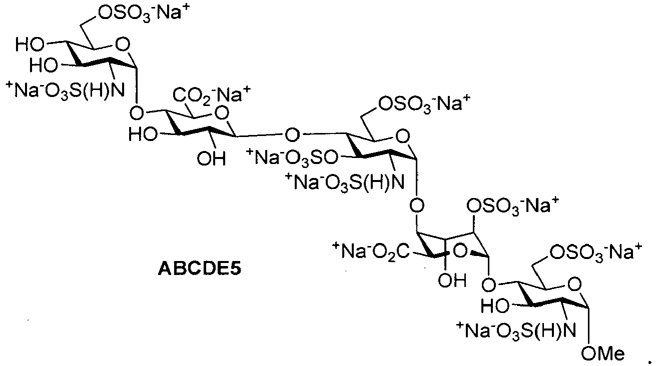

2. The process of claim 1, wherein the ratio of toluene/MTBE is about 20%.

3. The process of claim **1,** wherein step ii) further comprises
ii-1) converting the compound of formula **ABC1** in the presence of a promoter, an organic solvent, a base, and an acylating agent to a compound of formula **ABC2** 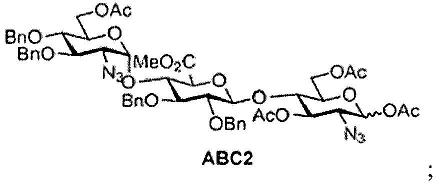
ii-2) converting the compound of formula **ABC2** in the presence of a base and an aprotic solvent to the compound of formula **ABC3** 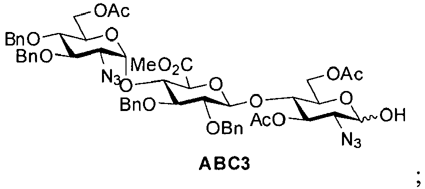
ii-3) converting the compound of formula **ABC3** in the presence of trichloracetonitrile and an aprotic solvent to the compound of formula **ABC4** 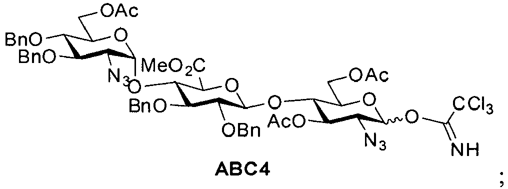
ii-4) converting the compound of formula **ABC4** in the presence of a promoter in an organic solvent and a thiol to provide the compound of formula **ABC5;** 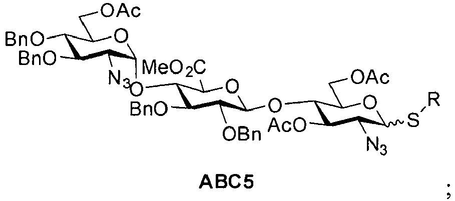
ii-5) contacting the compound of formula **ABC5** with the compound of formula **DE4** in the presence of a radical initiator and/or a promotor in an organic solvent 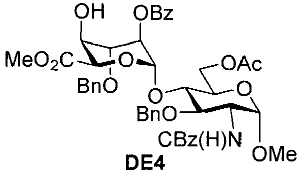 to obtain the compound of formula **ABCDE1** 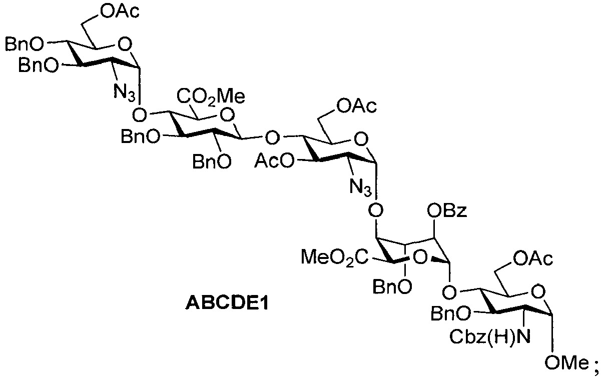
ii-6) converting the compound of formula **ABCDE1** in the presence of a peroxide and a base in an aprotic solvent to the compound of formula **ABCDE2** 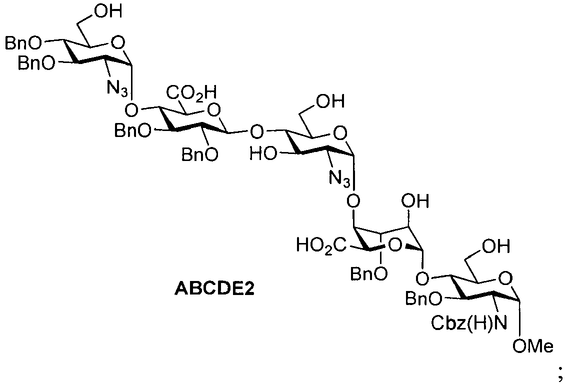
ii-7) converting the compound of formula **ABCDE2** in the presence of a sulfating agent in an aprotic solvent, followed by the addition of a base to the compound of formula **ABCDE3** 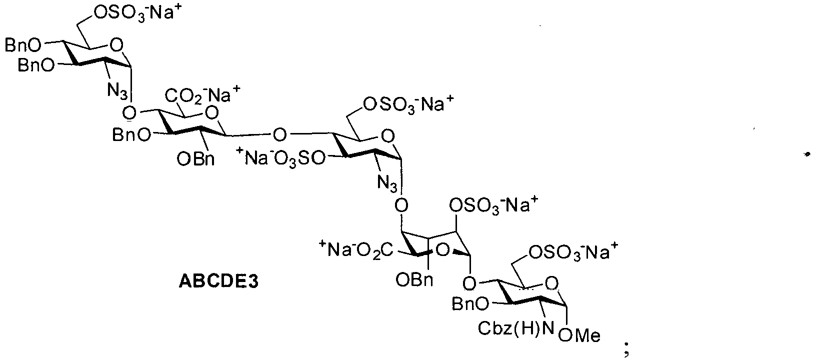
ii-8) converting the compound of formula **ABCDE3** in the presence of a hydrogenating catalyst and H₂ to the compound of formula **ABCDE4** 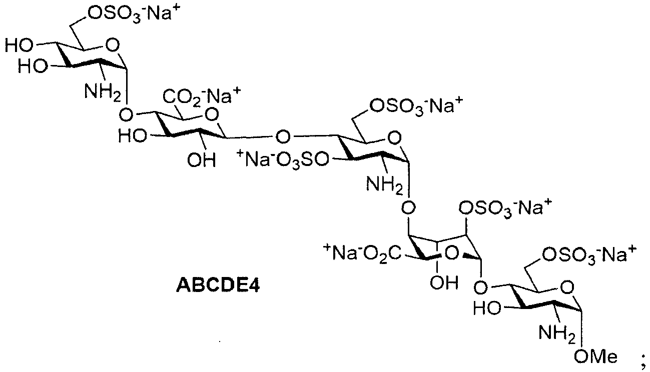
ii-9) converting the compound of formula **ABCDE4** to Fondaparinux sodium of formula **ABCDE5** using a sulfating reagent, followed by addition of a base 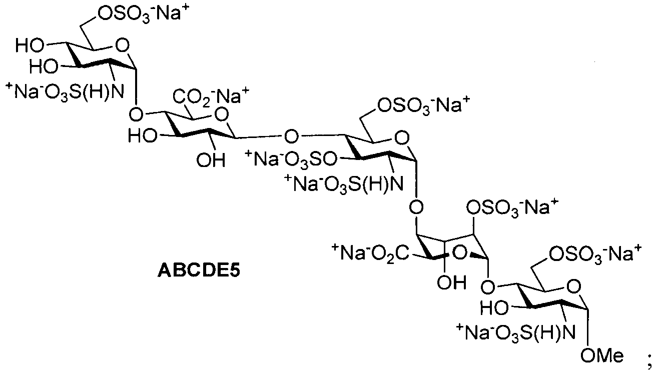 wherein
R is selected from the group consisting of alkyl, phenyl, benzyl, substituted alkyl, substituted phenyl, substituted benzyl.

4. The process of claim **1,** wherein step i) further comprises
a) converting a compound of formula **A4:** 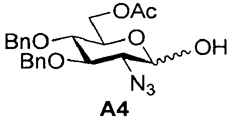 to a compound of formula **A5:**

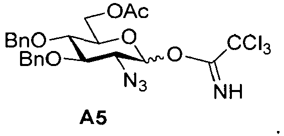

5. The process of claim **4,** wherein step a) is conducted in the presence of a base and trichloroacetonitrile.

6. The process of claim **5,** wherein the base is selected from the group consisting of DBU and potassium carbonate.

7. The process of claim **1,** wherein step i) is conducted in the presence of promoter.

8. The process of claim **3** or **7,** wherein the promoter is selected from the group consisting of triethylsilyl trifluoromethanesulfonate (TESOTf), trimethylsilyltrifluoromethanesulfonate (TMSOTf), tert-butyldimethylsilyl trifluoromethanesulfonate (TBSOTf), and mixtures thereof.

9. The process of claim **3,** wherein the radical initiator is selected from the group consisting of N-iodosuccinimide, and N-bromosuccinimide.

10. The process of claim **3,** wherein **ABC5** is **ABC5a:**

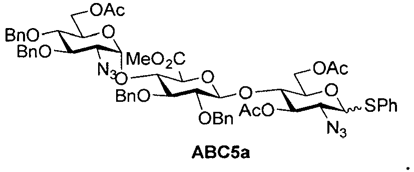

## Patentansprüche

1. Verfahren zur Herstellung von Fondaparinux-Natrium, welches umfasst:
i) Kontaktieren der Verbindung mit der Formel **A5:** 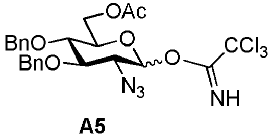 mit der Verbindung mit der Formel **BC8:** 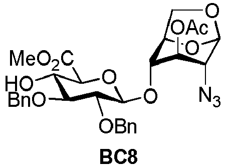 zum Bereitstellen der Verbindung mit der Formel **ABC1:** 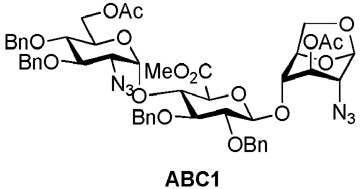 in einer Mischung von Toluol/MTBE; und
ii) Umwandeln der Verbindung mit der Formel **ABC1** in Fondaparinux mit der Formel **ABCDE5:**

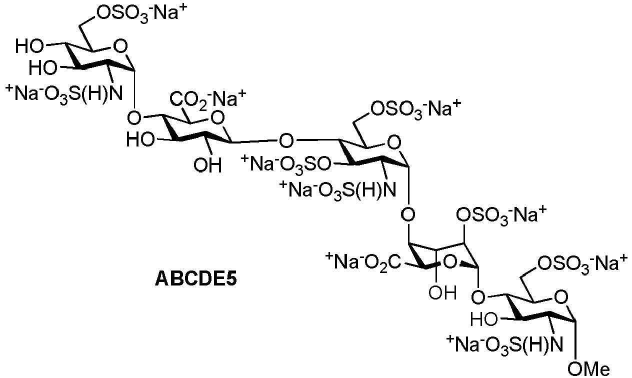

2. Verfahren nach Anspruch 1, wobei das Verhältnis von Toluol/MTBE etwa 20 % beträgt.

3. Verfahren nach Anspruch 1, wobei Schritt ii) ferner umfasst:
ii-1) Umwandeln der Verbindung mit der Formel **ABC1** in Gegenwart eines Promotors, eines organischen Lösungsmittels, einer Base und eines Acylierungsmittels in eine Verbindung mit der Formel **ABC2:** 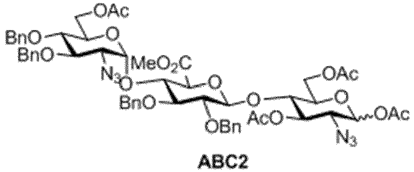
ii-2) Umwandeln der Verbindung mit der Formel **ABC2** in Gegenwart einer Base und eines aprotischen Lösungsmittels in eine Verbindung mit der Formel **ABC3:** 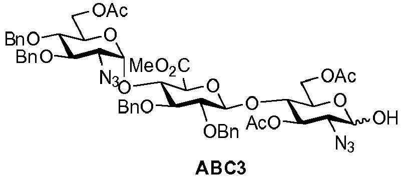
ii-3) Umwandeln der Verbindung mit der Formel **ABC3** in Gegenwart von Trichloracetonitril und einem aprotischen Lösungsmittel in eine Verbindung mit der Formel **ABC4:** 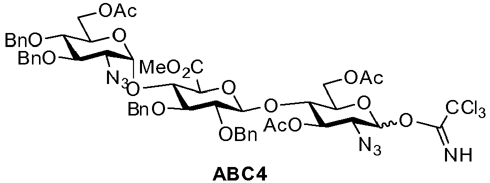
ii-4) Umwandeln der Verbindung mit der Formel **ABC4** in Gegenwart eines Promotors in einem organischen Lösungsmittel und eines Thiols zum Bereitstellen der Verbindung mit der Formel **ABC5:** 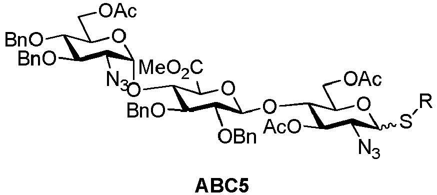
ii-5) Kontaktieren der Verbindung mit der Formel **ABC5** mit der Verbindung mit der Formel **DE4** in Gegenwart eines radikalischen Initiators und/oder eines Promotors in einem organischen Lösungsmittel 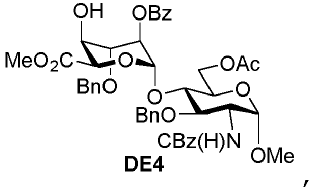 um die Verbindung mit der Formel **ABCDE1** zu erhalten: 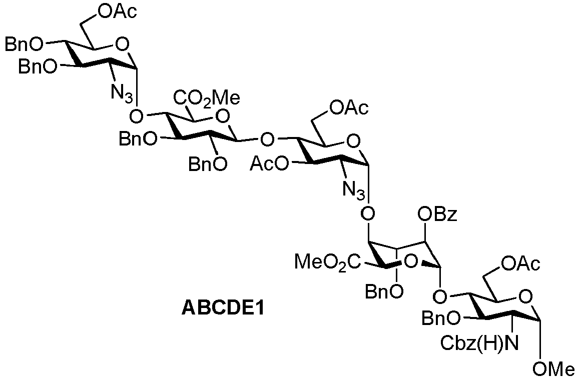
ii-6) Umwandeln der Verbindung mit der Formel **ABCDE1** in Gegenwart eines Peroxids und einer Base in einem aprotischen Lösungsmittel in eine Verbindung mit der Formel **ABCDE2:** 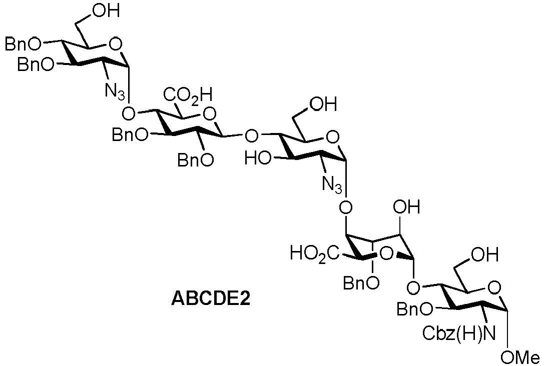
ii-7) Umwandeln der Verbindung mit der Formel **ABCDE2** in Gegenwart eines Sulfatierungsmittels in einem aprotischen Lösungsmittel, gefolgt von der Zugabe einer Base, in die Verbindung mit der Formel **ABCDE3:** 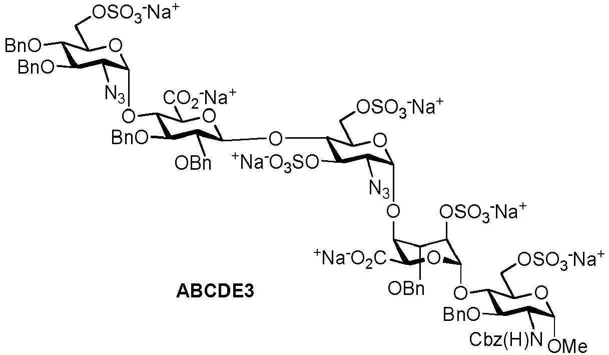
ii-8) Umwandeln der Verbindung mit der Formel **ABCDE3** in Gegenwart eines Hydrierkatalysators und H₂ in eine Verbindung mit der Formel **ABCDE4:** 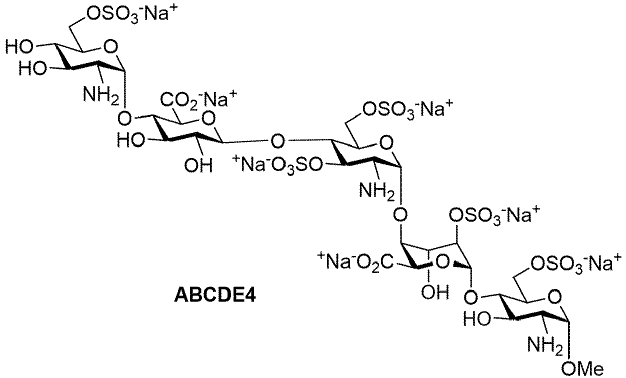
ii-9) Umwandeln der Verbindung mit der Formel **ABCDE4** in Fondaparinux-Natrium mit der Formel **ABCDE5** unter Verwendung eines Sulfatierungsreagenzes, gefolgt von der Zugabe einer Base: 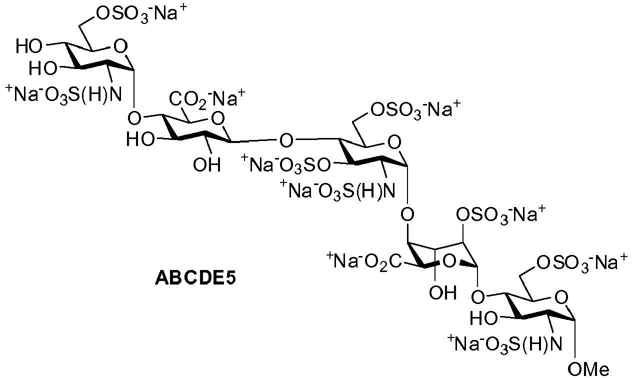 wobei
R ausgewählt ist aus der Gruppe bestehend aus Alkyl, Phenyl, Benzyl, substituiertem Alkyl, substituiertem Phenyl, substituiertem Benzyl.

4. Verfahren nach Anspruch 1, wobei Schritt i) ferner umfasst:
(a) Umwandeln einer Verbindung mit der Formel **A4:** 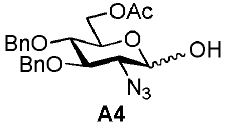 in eine Verbindung mit der Formel **A5:**

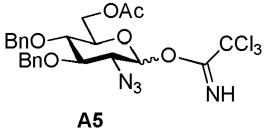

5. Verfahren nach Anspruch 4, wobei Schritt a) in Gegenwart von einer Base und Trichloracetonitril durchgeführt wird.

6. Verfahren nach Anspruch 5, wobei die Base ausgewählt ist aus der Gruppe bestehend aus DBU und Kaliumcarbonat.

7. Verfahren nach Anspruch 1, wobei Schritt i) in Gegenwart eines Promotors durchgeführt wird.

8. Verfahren nach Anspruch 3 oder 7, wobei der Promotor ausgewählt ist aus der Gruppe bestehend aus Triethylsilyltrifluormethansulfonat (TESOTf), Trimethylsilyltrifluormethansulfonat (TMSOTf), tert-Butyldimethylsilyltrifluormethansulfonat (TBSOTf) und Mischungen davon.

9. Verfahren nach Anspruch 3, wobei der radikalische Initiator ausgewählt ist aus der Gruppe bestehend aus N-Iodsuccinimid und N-Bromsuccinimid.

10. Verfahren nach Anspruch 3, wobei **ABC5 ABC5a** ist:

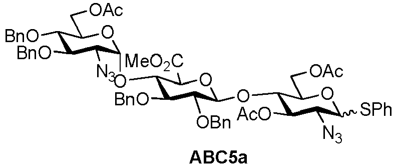

## Revendications

1. Procédé de préparation de sodium de Fondaparinux comprenant :
i) la mise en contact du composé de formule **A5** 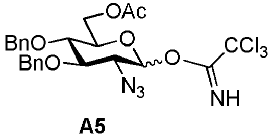 avec le composé de formule **BC8** 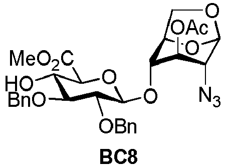 pour obtenir le composé de formule **ABC1** 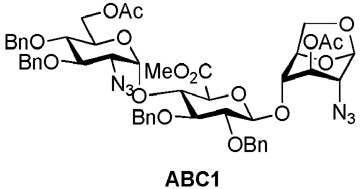 dans un mélange de toluène/MTBE ; et
ii) la conversion du composé de formule **ABC1** en Fondaparinux de formule **ABCDE5** :

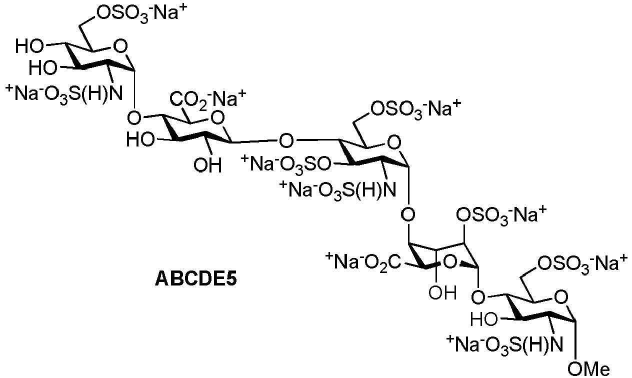

2. Procédé selon la revendication 1, dans lequel le rapport de toluène/MTBE est d'environ 20 %.

3. Procédé selon la revendication 1, dans lequel l'étape ii) comprend en outre
ii-1) la conversion du composé de formule **ABC1** en présence d'un promoteur, d'un solvant organique, d'une base et d'un agent d'acylation en un composé de formule **ABC2** 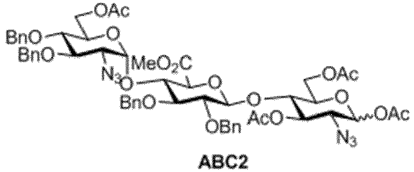
ii-2) la conversion du composé de formule **ABC2** en présence d'une base et d'un solvant aprotique en le composé de formule **ABC3** 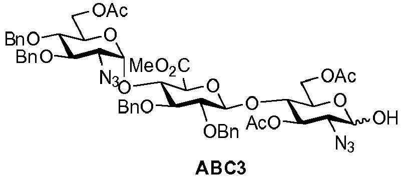
ii-3) la conversion du composé de formule **ABC3** en présence de trichloracétonitrile et d'un solvant aprotique en le composé de formule **ABC4** 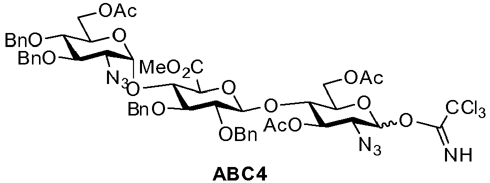
ii-4) la conversion du composé de formule **ABC4** en présence d'un promoteur dans un solvant organique et d'un thiol pour obtenir le composé de formule **ABC5** ; 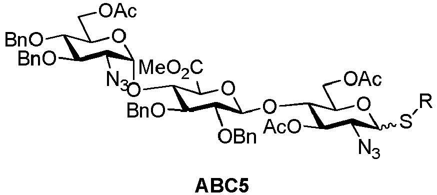
ii-5) la mise en contact du composé de formule **ABC5** avec le composé de formule **DE4** en présence d'un initiateur radicalaire et/ou d'un promoteur dans un solvant organique 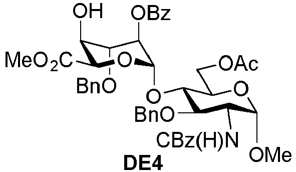 pour obtenir le composé de formule **ABCDE1** 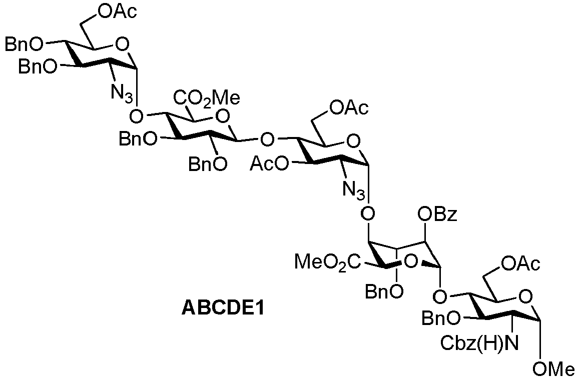
ii-6) la conversion du composé de formule **ABCDE1** en présence d'un peroxyde et d'une base dans un solvant aprotique en le composé de formule **ABCDE2** 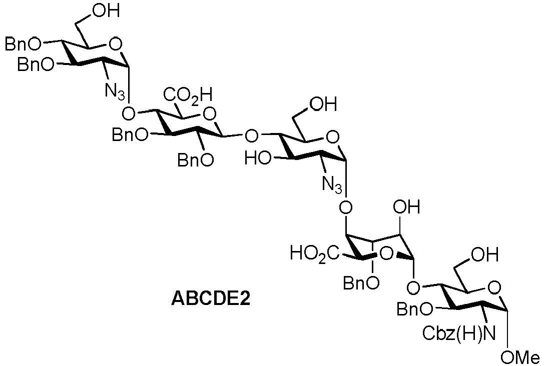
ii-7) la conversion du composé de formule **ABCDE2** en présence d'un agent de sulfatation dans un solvant aprotique, suivie par l'ajout d'une base, en le composé de formule **ABCDE3** 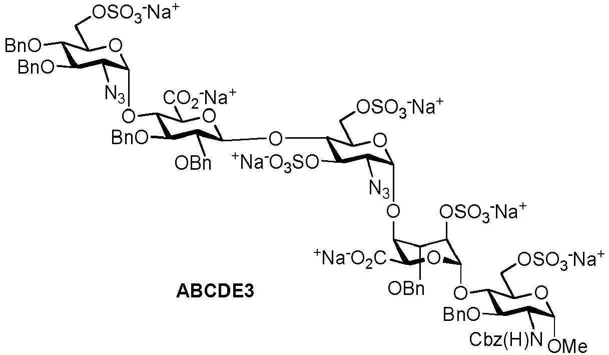
ii-8) la conversion du composé de formule **ABCDE3** en présence d'un catalyseur d'hydrogénation et de H₂ en le composé de formule **ABCDE4** 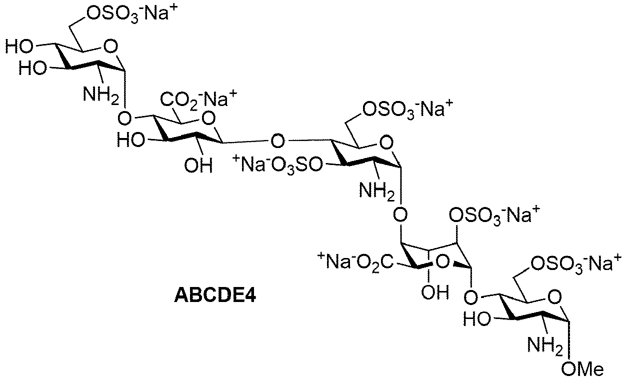
ii-9) la conversion du composé de formule **ABCDE4** en sodium de Fondaparinux de formule **ABCDE5** à l'aide d'un réactif de sulfatation, suivie par l'ajout d'une base 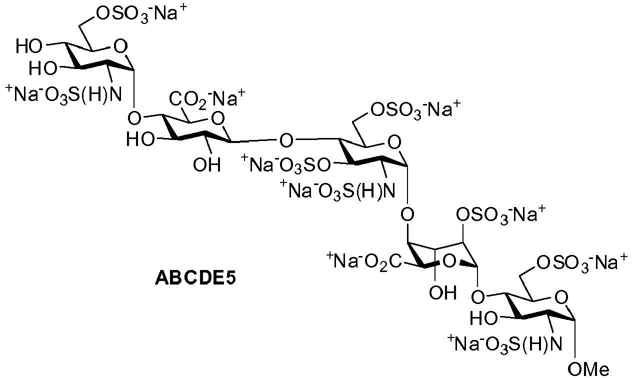 dans laquelle
R est choisi dans le groupe constitué par alkyle, phényle, benzyle, alkyle substitué, phényle substitué, benzyle substitué.

4. Procédé selon la revendication 1, dans lequel l'étape i) comprend en outre
a) la conversion d'un composé de formule **A4** : 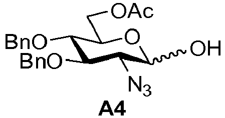 en un composé de formule **A5** :

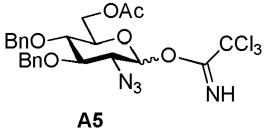

5. Procédé selon la revendication 4, dans lequel l'étape a) est conduite en présence d'une base et de trichloroacétonitrile.

6. Procédé selon la revendication 5, dans lequel la base est choisie dans le groupe constitué de DBU et de carbonate de potassium.

7. Procédé selon la revendication 1, dans lequel l'étape i) est conduite en présence d'un promoteur.

8. Procédé selon la revendication 3 ou 7, dans lequel le promoteur est choisi dans le groupe constitué par le trifluorométhanesulfonate de triéthylsilyle (TESOTf), le triméthylsilyltrifluorométhanesulfonate (TMSOTf), le tert-butyldiméthylsilyltrifluorométhanesulfonate (TBSOTf), et leurs mélanges.

9. Procédé selon la revendication 3, dans lequel l'initiateur radicalaire est choisi dans le groupe constitué par le N-iodosuccinimide et le N-bromosuccinimide.

10. Procédé selon la revendication 3, dans lequel **ABC5** est **ABC5a :**
